Europäisches Patentamt

(19) European Patent Office

Office européen des brevets

(11) Veröffentlichungsnummer : **0 205 512 B1**

(12) **EUROPÄISCHE PATENTSCHRIFT**

(45) Veröffentlichungstag der Patentschrift :
05.06.91 Patentblatt 91/23

(21) Anmeldenummer : 86900130.5

(22) Anmeldetag : 16.12.85

(86) Internationale Anmeldenummer :
PCT/EP85/00709

(87) Internationale Veröffentlichungsnummer :
**WO 86/03667 03.07.86 Gazette 86/14**

(51) Int. Cl.⁵ : **A61B 17/58, A61F 2/30**

(54) **VERSCHLUSS AUS CHIRURGISCHEM MATERIAL.**

(30) Priorität : **14.12.84 DE 3445709**

(43) Veröffentlichungstag der Anmeldung :
**30.12.86 Patentblatt 86/52**

(45) Bekanntmachung des Hinweises auf die
Patenterteilung :
**05.06.91 Patentblatt 91/23**

(84) Benannte Vertragsstaaten :
**AT BE CH DE FR GB IT LI LU NL SE**

(56) Entgegenhaltungen :
**EP-A- 0 011 809**
**EP-A- 0 016 906**
**CH-A- 611 794**
**DE-A- 1 185 332**
**DE-A- 2 205 808**
**DE-A- 2 742 128**
**DE-A- 3 125 268**

(56) Entgegenhaltungen :
DE-A- 3 134 152
DE-B- 2 620 890
FR-A- 1 046 920
FR-A- 2 306 672
GB-A- 2 025 238
GB-A- 2 054 383
US-A- 2 381 050
US-A- 3 842 825
US-A- 3 855 638

(73) Patentinhaber : **DRAENERT, Klaus, Dr.med.**
**Gabriel-Max-Strasse 3**
**W-8000 München 90 (DE)**

(72) Erfinder : **DRAENERT, Klaus, Dr.med.**
**Gabriel-Max-Strasse 3**
**W-8000 München 90 (DE)**

(74) Vertreter : **Vossius & Partner**
**Siebertstrasse 4 P.O. Box 86 07 67**
**W-8000 München 86 (DE)**

## Beschreibung

Gegenstand der Erfindung ist ein Verschluß aus chirurgischem Material zum Verschließen von Defekten oder Löchern eines Knochens und/oder einer Markhöhle.

Die Erfindung gilt einem Problem, welches bislang wenig Beachtung fand, aber von großer klinischer Tragweite ist. Wenn Schrauben, die zur Fixation einer Fraktur in den Knochen appliziert worden sind, nach Verheilung der Fraktur wieder entfernt werden, entstehen Defekte und/oder Löcher im Knochen. Es ist eine Erfahrungstatsache aus der klinischen Praxis, daß es aus diesen Schraubenlöchern sehr stark blutet und daß es notwendig ist, über 48 Stunden und länger eine Saugdrainage in die Wunde einzulegen, um Blutergüsse und die damit sehr häufig verbundenen Infektionen der Wunde und damit des Knochens zu vermeiden.

Gleichwohl gelang es selten, die Infektionsrate nach Metallentfernungen nach geheilten Frakturen prozentual so niedrig zu halten, wie dies nach der Erstversorgung der Frakturen, mit Ausnahme der offenen Frakturen, nach Einführung besonderer Luftreinigunssvorrichtungen im Operationssaal, wie laminar air flow, gelungen ist. Diesem Phänomen wurde bislang nur insofern Beachtung geschenkt, als man glaubte, daß die erhöhte Infektionsrate mit der Nachlässigkeit dieser als Bagatelleingriff angesehenen und abgestempelten Operation in Verbindung gebracht wurde. Es wurde daher letztlich gefordert, daß der Erstoperateur auch das Metall wieder entfernt. Gleichwohl liegt die Infektionsrate und die Rate der Komplikationen durch Nachblutung und Hämatome noch weit über der der Erstversorgung.

Auch bei Amputationsstümpfen traten, ohne daß die Ursache hierfür erkannt worden wäre, exzessive Hämatombildungen auf, die postoperativ in den ersten Tagen kaum zu beherrschen waren. Man versuchte zwar, diese Komplikationen durch Kompressionsverbände einzuschränken, die Zahl der infizierten Amputationsstümpfe ist jedoch sehr hoch, und sie konnte auch nicht durch die Anwendung des laminar air flow verringert werden. Bislang gelang es nicht, diese Blutergußbildungen und Infektionen sicher zu verhindern.

Aus FR-A-2 306 672 ist eine Amputationsstumpfplastik zum Abschirmen des scharfkantigen Knochenendes nach einer Amputation bekannt. Dabei wird darauf abgezielt, ein als "Hypertrophie" bezeichnetes Phänomen zu stoppen, das insbesondere bei Jugendlichen auftritt und bei dem das freiliegende Ende eines teilweise amputierten Knochens weiter wächst und ein scharfkantiges Knochenende bildet. Diese bekannte Amputationsstumpfplastik verschließt jedoch nicht hermetisch und druckdicht, sondern bewirkt lediglich ein mechanisches Begrenzen des Knochenwachstums und ist allenfalls nach Einwachsen des Knochens dicht.

Aus GB-A-2 054 383 ist ein Pfropfen für die Markhöhle eines Knochens bekannt, der in die Markhöhle eingeführt wird und als Barriere für den Knochenzement während des Einbringens einer Prothese dient. Der Pfropfen soll sich in der Markhöhle mechanisch verblocken, muß aber die Markhöhle nicht druckdicht abschließen, da Knochenzement sehr viskos ist und nach dem Einbringen schnell aushärtet.

Außerdem sind Kunststoffschrauben für die Osteosynthese bekannt, beispielsweise aus DE-A-27 42 128, die allerdings aufgrund ihrer Härte nicht geeignet sind, sich einem Knochendefekt anzupassen. Es sind somit bisher keine Verfahren oder Erzeugnisse bekannt, mit denen die genannten Hämatombildungen und Infektionen sicher verhindert werden können.

Der Erfindung liegt die Aufgabe zugrunde, ein Erzeugnis bereitzustellen, mit dem insbesondere infolge von operativen Eingriffen auftretende Hämatombildungen und Infektionen an Amputationsstümpfen und Defekten oder Löchern im Knochen verhindert werden können.

Diese Aufgabe wird durch den Verschluß gemäß den Patentansprüchen gelöst.

Die Erfindung geht dabei von dem Grundgedanken aus, Defekte und/oder Löcher eines Knochens und/oder einer Markhöhle gegenüber dem Innendruck der Knochenkanäle und/oder der Markhöhle mittels eines Verschlusses aus chirurgischem Material hermestisch bzw. druckdicht abzudichten.

Die in Lehrbüchern beschriebene Funktion des Knochens besteht in einer statischen und statisch-dynamischen Stützfunktion. Der Aufbau des Knochens wird demnach als ein System von Bälkchen- und Lamellensystemen beschrieben, welche sich nach den einwirkenden Kräften ausrichten und dimensionieren und welche daher auch Trajektorienbauweise aufweisen.

Bei einem solchen System müßte der Knochen in seiner Konstruktion eine Reservekapazität haben, die es ihm erlaubt, einer Beanspruchung bis zum zehnfachen seiner üblichen Stützleistung standzuhalten. Dies ist jedoch morphologisch in keinem Bereich des Knochens erkennbar.

Folglich wurde angenommen, daß der Körper andere Steuerungssysteme unterhält und daß die Festigkeit der Knochen auf anderen Prinzipien beruht.

Es wurde nun zufällig gefunden, daß die Blutung aus Bohrlöchern und Schraubenkanälen dann verstärkt war, wenn die Muskulatur der entsprechenden Extremität tonisiert wurde.

Die Hämatombildung nach Metallentfernung ist deswegen so ausgeprägt, da aufgrund der wieder intakten Funktion einer Gliedmaße Patienten, denen das Metall vom Knochen entfernt worden ist, diese Extremität wieder voll beanspruchen und die Muskulatur aktiv einsetzen. Bei diesen Patienten blutet es demnach stark aus den noch offenen Schraubenkanälen, so daß sich ein ausgedehntes Hämatom bis unter die Haut bilden kann.

Die vorstehenden Befunde können dadurch gedeutet werden, daß ein ganz wesentliches System zur Steuerung der Stabilität des Knochens im Gefäßsystem als solchem zu sehen ist. Es wird daher erfindungsgemäß davon ausgegangen, daß der Knochen ein hydraulisches System darstellt, das mit dem Prinzip des Innendrucks arbeitet.

Aus morphologischen Studien an Bohrlochdefekten ist ebenfalls zu vermuten, daß der Knochen ein abgeschlossenes System darstellt, in dem ein Druck aufgebaut werden kann.

Die erfindung basiert auf einer systematischen Erarbeitung des Gefäßsystemes und seiner Morphologie. Das Gefäßsystem des Knochens wurde mit Kunststoff ausgegossen und in Kombination mit Schnitt- und Serienschliffen die rasterelektronenmikroskopischen, dreidimensionalen Darstellungen des Gefäßsystems, zusammen mit dem Knochen, ergänzt. Diese Methode erlaubte die Auswertung und Analysierung des Gefäßsystems nach seinen einzelnen Abschnitten aufgrund der Abdrücke der Endothelien auf dem Kunststoffausguß.

Nach diesen Befunden stellt das Gefäßsystem des Knochens ein zentrifugales arterielles Durchströmungssystem dar, welches durch ein zentripetales venöses Drainagesystem ergänzt wird.

Ein Loch in der Markhöhle stellt in dem Moment die Quelle einer nicht stillbaren Blutung dar, wo die Muskulatur anfängt, sich zu tonisieren oder gar normal zu arbeiten.

Genau dies ist jedoch bei Metallentfernungen und Schraubenentfernungen infolge der Behandlung eines Knochendefekts der Fall. Bei geheilter Fraktur und funktionsfähigen Gliedmaßen wird das Metall herausgeschraubt und die Löcher im Knochen bleiben offen. Der Patient erhält seine volle Muskelfunktion und die Folge sind ausgedehnte Hämatome. Die Komplikation der Hämatome stellt die Superinfektion dieser Retention dar.

Dies erklärt letztlich die hohe Infektionsrate und hohe Komplikationsrate nach Metallentfernung, insbesondere in den Knochenabschnitten, in denen der Knochen und damit die Knochendefekte direkt unter der Haut liegen. Diese Zusammenhänge waren bisher nicht erkannt worden, und aus diesem Grunde war auch keine direkte Therapie möglich gewesen. Die empirische Anwendung der Saugdrainage bei Metallentfernungen über 24 bzw. 48 und manchmal auch 72 Stunden sollte diesem mißlichen Phänomen der Nachblutung nach Metallentfernungen Rechnung tragen.

Aufgrund der der Erfindung zugrunde liegenden Erkenntnisse werden mit dem erfindungsgemäßen Verschluß die Knochenöffnungen primär verschlossen und hermetisch abgedichtet, so daß sich im Inneren des Knochens oder der Markhöhle wie bei einem gesunden, defektfreien Knochen ein Innendruck aufbauen kann.

Bei einem direkten Vergleich des nicht verschlossenen und verschlossenen Knochendefektes bei funktioneller Nachbehandlung zeigt sich, daß der erfindungsgemäße Verschluß eine Veränderung der Durchblutung mit großer Effizienz erreichen kann und weitreichende sowie tragische Komplikationen für den Patienten vermieden werden können. Die Vorteile des erfindungsgemäßen Verschlusses liegen somit darin, daß zuverlässig die Komplikationen postoperativer Hämatombildung mit sukzessiver Superinfektion vermieden werden.

In einer bevorzugten Ausführungsform der Erfindung ist der Verschluß aus chirurgischem Material zur Abdichtung einkortikaler Defekte der Markhöhle ein zylindrischer oder im wesentlichen kegelstumpfförmiger Pfropfen, gegebenenfalls mit etwa halbkugelförmigem Aufsatz, ein Deckel, eine oder mehrere Kugeln oder ein aus einer Platte oder Folie, die vorzugsweise aus einem resorbierbaren Kunststoff, wie Kollagen-Apatit oder Polyglykolat-Apatit bestehen, gewickelter Lamellenzylinder. Derartige Verschlüsse können auch nach Entfernen des Marknagels in die verbleibende Öffnung eingepaßt werden.

In einer weiteren bevorzugten Ausführungsform ist der Verschluß zur Abdichtung von Schraubenkanälen eine Schraube, vorzugsweise aus resorbierbarem Polyglykolat-Apatit. Ein geeignetes Schraubensortiment kann in Anlehnung an die Maße und Gewindeformen der entsprechenden, im Handel erhältlichen Metallschrauben zusammengestellt werden. Durch Eindrehen der erfindungsgemäßen Schraube nach dem operativen Entfernen der Metallschraube läßt sich der Schraubenkanal auf besonders einfache und zuverlässige Weise druckdicht abschließen. Der erfindungsgemäße Verschluß kann auch als Schraube in Verbindung mit einem Deckel ausgebildet sein.

In einer anderen bevorzugten Ausführungsform ist der Verschluß derart ausgebildet, daß er die Markhöhle von Amputationsstümpfen gegen Innendrücke hermetisch abdichtet. Dabei muß der Verschluß so konstruiert sein, daß er eine stabile Fixation gewährleistet und der biomechanischen Beanspruchung des Amputationsstumpfes Rechnung trägt, beispielsweise als im Stumpf mechanisch zu verblockender Konus oder als durch Quellung fixierter Pfropfen oder Lamellenzylinder. Ein derartiger Verschluß stellt praktisch eine Amputationsstumpfendoprothese dar.

Vorzugsweise ist der erfindungsgemäße Verschluß zur Erleichterung der Verknöcherung aus Strukturen erster bis vierter Ordnung aufgebaut. Dabei gibt die Struktur erster Ordnung die äußere Form des Verschlusses an. Die Struktur zweiter Ordnung gibt Inhomogenitäten der Struktur erster Ordnung an, z.B. nimmt die Elastizität eines Verschlusses der Markhöhle eines Amputationsstumpfes vorzugsweise gegen die Markhöhle gerichtet kontinuierlich zu. Die Struktur dritter Ordnung ist die Oberflächenstruktur des Verschlusses. Eine bevorzugte Struktur dritter Ordnung weist einzelne Formelemente auf, die jeweils mit den benachbarten Formelementen in Kontakt sind und in einer oder mehreren miteinander in Kontakt stehenden Schichten angeordnet sind. Die einzelnen Formelemente stellen vorzugsweise Kugeln oder Kugelabschnitte, wie Halbkugeln mit einem Durchmesser von 200 bis 3000 μm dar.

Zu einem beschleunigten Knocheneinwuchs kommt es insbesondere dann, wenn größere Kugeln mit kleineren Kugeln kombiniert werden. Ein Durchmesserverhältnis von 2 : 1 bis 3 : 1 ist besonders günstig. Beispielsweise können Kugeln mit einem Durchmesser von 200 bis 1000 μm, im Mittel 500 μm, mit Kugeln mit einem Durchmesser von 800 bis 3000 μm, im Mittel 1000 μm, vermischt sein. Vorzugsweise weisen die kleineren Kugeln einen Durchmesser von 300 bis 700 μm, besonders bevorzugt 450-550 μm, und die größeren Kugeln einen Durchmesser von 800 bis 2000 μm, besonders bevorzugt 900-1200 μm auf. Dabei weisen die kleineren Kugeln einerseits und die größeren Kugeln andererseits vorzugsweise alle etwa den gleichen Durchmesser, also z.B. 500 μm und 1000 μm auf. Um die kleineren Kugelelemente kommt es schneller zu einer Knochenanlagerung, während die tragenden Knochengewölbe sich um die großen Kugelelemente ausbilden.

Die Oberflächen der Kugeln oder Kugelabschnitte können mikrostrukturiert sein, wobei die Mikrostrukturierung z.B. in Form von Kugeln oder Kugelabschnitten, wie Halbkugeln mit einem Durchmesser von 10 bis 50 μm, vorzugsweise 15 bis 30 μm; ausgebildet ist.

Die Zwischenräume zwischen den größeren Kugeln oder Wülsten und/oder die Oberfläche des Verschlusses können mit einer Beschichtungsmasse ausgefüllt bzw. bedeckt sein, die zusammen mit einer etwaigen Mikrostrukturierung der größeren Kugeln die Struktur IV. Ordnung des erfindungsgemäßen Verchlusses darstellt, Die Beschichtungsmasse besteht dabei vorzugsweise aus einer organischen, meist resorbierbaren Matrix und anorganischen Füllerbestandteilen oder Füllkörpern.

Die Außenseite des Verschlusses kann entweder eine dicke Beschichtung mit einer Dicke von etwa 300 bis 2.000 μm oder eine dünne Beschichtung mit einer Dicke von etwa 20 bis 300 μm aufweisen. Die Schicht kann entweder geschlossen sein und den Verschluß vollständig umfassen oder nur Teile des Verschlusses bedecken. Sie kann entweder direkt oder indirekt in einem Verbund aufgebracht sein.

Die Füllkörper bestehen vorzugsweise aus Kugelpartikeln mit einem Durchmesser von 10 bis 200 μm, vorzugsweise 15 bis 50 μm, besonders bevorzugt 15 bis 30 μm. Insbesondere die größeren Partikel sind zur Erleichterung des Einwachsens des Knochens vorzugsweise porös und weisen ein Porenvolumen von etwa 25 bis 65% auf. Die gennanten Kugeln werden von Osteoblasten als Basis erkannt und regen den Knocheneinwuchs an.

Die erfindungsgemäßen Verschlüsse werden vorzugsweise aus einem resorbierbaren oder nicht resorbierbaren, organischen und/oder anorganischen Material hergestellt. Besonders bevorzugt sind organische, resorbierbare Materialien, nachstehend auch "Biomaterialien" genannt, wie Polylactat, Polyglykolat oder Kollagen. Diese Materialien können sowohl für die Formelemente der Struktur dritter Ordnung als auch als Bindemittel oder Matrix für die Struktur vierter Ordnung oder Beschichtung verwendet werden. Zumindest in der Beschichtung sind Füllkörper aus einer härteren, resorbierbaren Substanz auf der Basis fester Calciumverbindungen, wie Apatit, vorzugsweise Hydroxylapatit, oder Tricalciumphosphat oder Bioglas eingelagert.

Die Matrix der Beschichtung, die auch die Poren der hochporösen Füllkörper auffüllen kann, besteht vorzugsweise aus einem Polypeptid, Polylactat, Polyglykolat oder einem deren Cokondensate, Gelatine, Kollagen und/oder Calciumverbindungen.

Als resorbierbare Materialien für die Formelemente oder Elementar-Körper der Struktur dritter Ordnung sind insbesondere Tricalciumphosphat, Apatit, wie Hydroxylapatit, ein Polypeptid, Kollagen oder Gemische dieser Materialien geeignet. Die Formelemente können aber auch aus einem Kunststoff oder einem Metall, wie Edelstahl, Titan, Tantal, Cobalt, Chrom, Molybdän oder deren Legierungen bestehen. Die resorbierbaren Schrauben haben ihre Indikation insbesondere dort, wo die mechanische Festigkeit des Verschlusses nicht im Vordergrund steht. Vorzugsweise wird mindestens die Beschichtung des Verschlusses aus quellfähigem Material hergestellt, das nach seinem Einbringen sein Volumen vergrößert und einen besonders dichten Anschluß gewährleistet. Zur Unterdrückung des Infektionsrisikos kann dem Material des Verschlusses vorzugsweise ein Antibiotikum, ein Chemotherapeutikum oder eine biologisch aktive Substanz zugesetzt sein.

Zumindest ein Teil der Füllkörper der Beschichtungsmasse kann in Form von Fasern mit einer Dicke von 100 bis 300 μm, vorzugsweise etwa 200 μm und einer Länge von vorzugsweise mehr als 2 bis 15 mm ausgebildet sein, wobei die bevorzugte Länge mindestens 3 mm und höchstens 10 mm und die optimale Länge etwa 4 bis 5 mm ist. Die faserförmigen Füllkörper verbessern die Stabilität und bestehen vorzugsweise aus den gleichen Materialien wie die kugelförmigen Füllkörper.

In der Beschichtung kann ebenfalls zusätzlich ein pharmazeutischer Wirkstoff, beispielsweise ein Antibiotikum enthalten sein.

Die erfindungsgemäßen Verschlüsse haben insbesondere zwei Indikationen. Bei einer funktionstüchtigen Extremität, die nicht mehr ruhiggestellt wird, muß ein Schraubenkanal- oder ein Bohrlochdefekt nach dem Entfernen der Kortikalis- oder Spongiosaschrauben primär wieder mit dem erfindungsgemäßen Verschluß verschlossen werden, um Nachblutungen und damit die Infektionsgefahr auszuschließen. Derartige Schrauben werden allein in der BRD jährlich millionenfach eingesetzt. Ebenso müssen aufgrund der Erkenntnis der Zusammenhänge zwischen der Knochendurchblutung und der Blutung aus dem Knochen Amputationsstümpfe mit den erfindungsgemäßen Verschlüssen hermetisch gegen den Innendruckaufbau der Markhöhle abgeschlossen werden. Bislang gab es dagegen allenfalls Versuche, Amputationsstumpfplastiken mit Knochen aus autologem und homologem Material herzustellen, die aber alle nur ein Ziel hatten, nämlich die scharfkantigen Knochenenden des Amputationsstumpfes gegen die weiche Muskulatur plastisch zu formen.

Für beide vorstehenden Indikationen eignen sich hervorragend die erfindungsgemäßen Verschlüsse aus einem Biomaterial auf der Basis des Kollagen-Apatites oder Glykolat-Apatites mit einem Antibiotikum, einem Chemotherapeutikum oder einer biologisch aktiven Substanz.

Die Erfindung wird nachstehend anhand der Zeichnung näher erläutert. Es zeigen :

Figur 1 einen erfindungsgemäßen Verschluß in Form einer Schraube zum Verschließen von zwei Schraubenlöchern in einem Knochen,

Figur 2 zwei erfindungsgemäße Verschlüsse in Form eines Zylinders,

Figur 3 einen erfindungsgemäßen Verschluß eines Knochendefekts in Form eines Lamellenzylinders,

Figur 4 einen erfindungsgemäßen Verschluß eines Amputationsstumpfes.

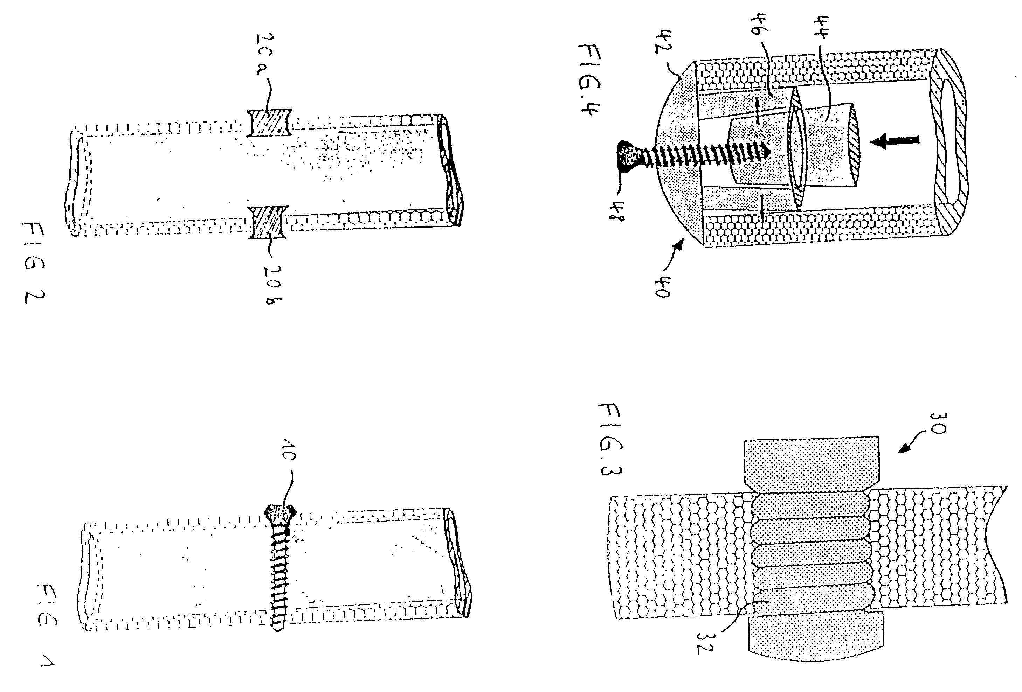

Beim Verschluß gemäß Figur 1 wird eine Schraube 10, vorzugsweise aus einem resorbierbaren Material, in den alten Schraubenkanal der zur Knochenfixation verwendeten AO-Schraube eingedreht und dichtet die Markhöhle hermetisch ab.

Gemäß Figur 2 werden zwei etwa zylinderförmige Pfropfen oder Stöpsel 20a und 20b in einen Knochendefekt, beispielsweise einen Schraubenkanal, eingedrückt. Die Stöpsel quellen im Kontakt mit der Körperflüssigkeit auf, dichten die Markhöhle ab und verformen sich etwa in Form eines Korkens.

Gemäß Figur 3 wird ein Lamellenzylinder 30 mit mehreren Lamellen 32 durch ein Loch in der als Bienenwabenstruktur angedeuteten Knochenröhre eingedrückt und verschließt, insbesondere nach ihrem Aufquellen, die Markhöhle hermetisch.

Der Verschluß 40 für einen Amputationsstumpf gemäß Figur 4 weist einen nahezu halbkugelförmigen oder pilzkopfförmigen Abschluß, bzw. eine Kappe 42, einen massiven, konusförmigen inneren Pfropfen 44, eine konusförmige Hülse 46 und eine Schraube 48 auf. Nach dem Einführen des Verschlusses 40 in den Amputationsstumpf wird durch Drehen der Schraube 48 der Pfropfen 44 in die Hülse 46 hineingezogen und verblockt die Hülse 46 mit der als Bienenwabenstruktur angedeuteten Knochenröhre. Dadurch wird der Amputationsstumpf hermetisch gegen seinen Innendruck abgeschlossen, Hämatombildungen und Infektionen verhindert und die Heilung beschleunigt.

Anstelle des Pfropfens 44 und der Hülse 46 gemäß der Ausführungsform von Figur 4 kann auch ein Zylinder oder Lamellenzylinder vorgesehen sein, der durch Drehen einer entsprechend Figur 4 angeordneten und eine Kontermutter aufweisenden Schraube gestaucht wird, dadurch seinen Durchmesser verbreitert und mit der Innenseite der Knochenröhre verblockt wird. Der Verschluß kann auch einfach etwa pilzförmig ausgebildet sein, wobei der "Stiel" des Pilzes in den Amputationsstumpf eingeführt wird und lediglich durch seine Quellung verblockt wird. Der Verschluß gemäß Fig. 4 und die vorstehend erläuterten Ausführungsformen stellen praktisch eine Amputationsstumpfprothese dar.

Die Beispiele erläutern die Erfindung.

**Beispiel 1**

Verschluß eines Schraubenkanals

Tierexperimentelle Versuche mit Eröffnung der Markhöhle und dem Vergleich des mit einem Stöpsel verschlossenen Defektes einerseits mit dem nicht verschlossenen Defekt auf der Gegenseite, unter funktioneller Nachbehandlung, zeigen eindrucksvoll die ausgedehnten Hämatome auf der Seite, bei der die Markhöhle nicht wieder verschlossen wurde.

Wenn der Schraubenkanal der Defekt oder das Bohrloch mit einem kleinen Stöpsel (vgl. Fig. 2) verschlossen wurde, blieb die Wunde vollständig bluttrocken. Die histologischen Untersuchungen zeigten, daß die Revaskularisation des Knochens auf der Seite, bei der die Markhöhle durch einen Pfröpfen oder Stöpsel verschlossen war, sehr viel schneller wieder ad integrum verheilte, wie auf der kontralateralen Seite, bei der das Bohr- und Defekt- loch offen blieb. 15 Tage nach der Operation war der Stöpsel vollständig knöchern integriert. Dieser Stöpsel war dabei z.B. als kleiner Lamellenzylinder, der in den Schraubenkanal eingedrückt wurde, ausgebildet.

In weiteren Experimenten wurde eine Kunststoffschraube oder eine Schraube (vgl. Fig. 1) aus einem resorbierbaren Material in den alten Schraubenkanal eingedreht, so daß dieser hermetisch auf einer Seite von innen nach außen und auf der anderen Seite von außen nach innen abgedichtet war. Dabei enthielten beide Schraubentypen ein Antibiotikum. Auch nach diesem operativen Eingriff erfolgte weder eine Hämatombildung, noch eine Infektion.

**Beispiel 2**

Verschluß eines Amputationsstumpfes

Der Verschluß von Amputationsstümpfen ist wesentlich, da aufgrund der Durchblutungsverhältnisse des Stumpfes der Stumpf so lange in die umgebenden Weichteile blutet, als die Markhöhle nicht abgedeckelt ist. Da die Durchmesser hierbei meist mehrere Zentimeter betragen, ist vor 2 bis 3 Wochen nicht mit einer Abdeckelung zu rechnen.

Der Verschluß der Amputationsstümpfe mit einem zylinderförmigen, mit Lamellen versehenen Markhöhlenverschluß gestattet eine hermetische Abdichtung gegenüber der Außenseite des Knochens, so daß sich in der Markhöhle die gewohnten Drücke aufbauen können und die Revaskularisation des Knochenstumpfes sehr schnell vonstatten geht.

Die Verschlüsse offener Amputationsstümpfe des Knochens konnten in optimaler Weise so gestaltet werden, daß ein Gewinde eines sogenannten "Lamellenzylinders" (vgl. z.B. auch Fig. 3) die Markhöhle nach außen abdichtete und/oder daß ein halbkugelförmig ausgebildeter Abschluß die freien Enden des Kompaktaknochens überragte.

Die Knochenbildung am Prothesenstumpf war dann besonders schnell, wenn die formgebenden Elemente des Amputationsverschlusses halbkugelförmige Gebilde mit einem Durchmesser von etwa 200-300 µm, vorzugsweise etwa 500 bis 2000 µm waren, die in sich wieder auf Zellbasis mikrostrukturiert waren, in der Form, daß kleine Halbkugeln eines Durchmessers von 10-200 µm, vorzugsweise 20 bis 40 µm diese Mikrostruktur der großen Kugeln ausmachten. Eine solche Strukturierung kann durch Beschichtung mit organischen Materialien, welche mit kugelförmigen Apatiten bestückt sind, erreicht werden.

Die Markhöhlenverschlüsse konnten vorteilhafterweise aber auch durch selbstverblockende Systeme stabil und frei von Oberflächenverschiebungen im Knochen fixiert werden. Dabei wurden entweder zwei konische Verschlüsse (vgl. Fig. 4) durch eine eingeführte Schraube ineinandergepreßt und dann die Querschnittsfläche vergrößert oder ein Kugelkonglomerat wurde in der Markhöhle des Knochens durch eine zentrale Schraube verblockt. Immer kam es bei hermetischem Abschluß der Markhöhle zum schnellen knöchernen Durchwaschsen des Markhöhlenverschlusses und zur totalen Verknöcherung des Amputationsstumpfes. Die Weichteile des Amputationsstumrfes blieben frei von Hämatomen, und die Infektionsrate konnte unter 1% gesenkt werden.

Da die biomechanische Beanspruchung des Amputationsstumpfes gegenüber der Beanspruchung der freien Gelenkenden der Diarthrosen stark zurücktritt, wurden für diese Markhöhlenverschlüsse auch resorbierbare Amputationsstumpfverschlüsse bzw. -prothesen auf der Basis der vorstehenden Biomaterialien erfolgreich eingesetzt.

Die Herstellung eines Verschlusses für einen Amputationsstumpf kann folgendermaßen durchgeführt werden :

a) In einem Schmelztiegel werden 5 g Polyglykolat-Granulat mit einigen Gramm bioverträglichem Weichmacher bei einer Temperatur von ca. 130°C geschmolzen. In die heiße Schmelze gibt man 1 g fein pulverisiertes Tricalciumphosphat und rührt solange, bis die Schmelze homogen ist. Die heiße Schmelze gießt man anschließend in eine Metallform und läßt sie erkalten. Die Metallform wird der Einfachheit halber durch Ausfräsen der gewünschten Oberflächenstrukturen aus einem Metall- oder Kunststoffblock hergestellt. Zweckmäßigerweise besteht die Gußform aus zwei symmetrischen Hälften, die nach Eingießen der heißen Schmelze zusammengepreßt und die nach Erkalten der Masse wieder getrennt werden.

b) Als Alternative zu Beispiel a) bietet sich die Herstellung des Amputationsstumpfes mittels Spritzgußverfahren an. Hierzu wird eine Schmelze aus Polyglykolaten, bioverträglichem Weichmacher und Tricalciumphosphat in einer eigens dafür vorgesehenen Apparatur hergestellt und anschließend mittels eines Druckes von ca. 20 bar in eine Spritzgußform eingespritzt. Nach Erkalten der Schmelze wird die Form zerlegt und der fertige Amputationsstumpf-Verschluß von der Form befreit.

**Ansprüche**

1. Verschluß aus chirurgischem Material zum Verschließen von Defekten oder Löchern eines Knochens und/oder einer Markhöhle, welcher derart ausgebildet ist, daß er eine solche Form aufweist und/oder aus einem Material gebildet ist, so daß er sich an die Abmessungen der Defekte oder Löcher des Knochens und/oder an die Abmessungen der Markhöhle anpaßt und daß dadurch die Defekte oder Löcher des Knochens und/oder die Markhöhle gegenüber dem Innendruck der Knochenkanäle und/oder der Markhöhle druckdicht abdichtbar sind.

2. Verschluß nach Anspruch 1, dadurch gekennzeichnet, daß er in Form einer Schraube (10), eines Dübels, eines Konus (40), eines Zylinders, eines Lamellenzylinders (30), eines Deckels, eines Pfropfens (20), eines Kugelverschlusses, eines Körbchenimplantats oder eines Hohlzylinderimplantats ausgebildet ist, und/oder daß er von außen nach innen zum Markraum gerichtet und/oder von innen nach außen vom Markraum weggerichtet applizierbar, schraubbar und/oder verblockbar ist.

3. Verschluß nach Anspruch 2 in Form einer Schraube (10), dadurch gekennzeichnet, daß deren Abmessungen den Abmessungen des Schraubenkanals einer vorher zur Behandlung einer Knochenfraktur verwendeten Metallschraube entsprechen.

4. Verschluß nach Anspruch 1 bis 3, dadurch gekennzeichnet, daß er derart ausgebildet ist, daß er die Markhöhle von Amputationsstümpfen gegen Innendrücke hermetisch abdichtet, und vorzugsweise als Kappe über das freie Ende eines Marknagels ausgebildet ist.

5. Verschluß nach Anspruch 1 bis 4, dadurch gekennzeichnet, daß er zur Erleichterung der Verknöcherung eine Struktur erster bis vierter Ordnung aufweist, wobei die Stuktur erster Ordnung die äußere Form darstellt, die an die Abmessungen des zu verschließenden Defektes oder Loches angepaßt ist, die Struktur zweiter Ordnung derart ausgebildet ist, daß die Elastizität des Verschlusses vom Amputationstumpf gegen die Markhöhle zu kontinuierlich zunimmt, so daß sich seine einzelnen Abschnitte an das Knochengerüst anpassen, und die die Oberflächenstruktur darstellende Struktur dritter Ordnung einzelne Formelemente aufweist, die jeweils mit den benachbarten Formelementen in Kontakt sind und in einer oder mehreren miteinander in Kontakt stehenden Schichten angeordnet sind.

6. Verschluß nach Anspruch 5, dadurch gekennzeichnet, daß die Formelemente Kugeln und/oder Wülste oder Schläuche mit einem Durchmesser von 200 bis 3000 μm sind, die vorzugsweise an ihrer Oberfläche mikrostrukturiert sind, wobei die Mikrostruktur vorzugsweise Kugelabschnitte oder Kugeln mit einem Durchmesser von 10 bis 200 μm, vorzugsweise 15-30 μm aufweist, und/oder aus Kugeln in einem Verbund mit einer Matrixschicht besteht, wobei als Matrix ein organisches Material auf der Basis eines Kollagens, Polyglykolates, Polylactates oder eines Polypeptids verwendet wird.

7. Verschluß nach Anspruch 1 bis 6, dadurch gekennzeichnet, daß er aus einem resorbierbaren und/oder nicht resorbierbaren organischen und/oder anorganischen Material besteht.

8. Verschluß nach Anspruch 5 bis 7, dadurch gekennzeichnet, daß die Formelemente aus Tricalciumphosphat, Apatit, Kollagen, einem Polypeptid oder einem Gemisch hieraus und/oder aus einem Metall, wie Titan, Tantal, Cobalt, Chrom, Molybdän oder einer ihrer Legierungen und/oder aus Edelstahl bestehen.

9. Verschluß nach Anspruch 1 bis 8 gekennzeichnet durch eine Beschichtung aus anorganischen Füllkörpern und einer organischen Matrix, die vorzugsweise die Zwischenräume zwischen den Formelementen ausfüllt und/oder die Oberfläche des Verschlusses bedeckt, wobei die Füllkörper der Beschichtung vorzugsweise Kugeln mit einem Durchmesser von 10 bis 200 μm, vorzugsweise 15 bis 30 μm sind, und/oder hochporöse Partikel aus Tricalciumphosphat oder Hydroxylapatit oder Partikel aus einer verwandten Calciumverbindung oder aus Bioglas, und wobei die Matrix der Beschichtung vorzugsweise aus einem Polypeptid, Kollagen, Polyglykolat oder Polylactat besteht.

10. Verschluß nach Anspruch 9, dadurch gekennzeichnet, daß die Füllkörper der Beschichtung zum Teil Faserform haben, und/oder ein geschlossenes Netz bilden.

11. Verschluß nach einem der Ansprüche 1 bis 10, dadurch gekennzeichnet, daß er selbst und/oder seine Beschichtung aus quellfähigem Material besteht.

12. Verschluß nach den Ansprüchen 1 bis 11, dadurch gekennzeichnet, daß im Verschluß und/oder in seiner Beschichtung ein pharmazeutischer Wirkstoff enthalten ist, z.B. ein Antibiotikum wie, Gentamycin, und/oder ein Zytostatikum und/oder "Bone morphogenetic Protein" und/oder Hämostyptikum.

## Claims

1. A closure of surgical material to close defects or holes in bone and/or a medullary cavity, which is formed in such a way that it has such a shape and/or is made of a material so that it conforms to the dimensions of the defects or holes in the bone and/or to the dimensions of the medullary cavity and that thereby the defects or holes in the bone and/or the medullary cavity are sealable in a pressure-tight manner vis-à-vis the internal pressure of the bone canals and/or the medullary cavity.

2. The closure according to claim 1, characterized in that it takes the form of a screw (10), a dowel, a cone (40), a cylinder, a lamellar cylinder (30), a cap, a stopper (20), a spherical valve, a cage implant or a hollow cylinder implant, and/or in that it can be applied, screwed in and/or interlocked from the outside to the inside towards the medullary space and/or from the inside to the outside away from the medullary space.

3. The closure according to claim 2 taking the shape of a screw (10), characterized in that its dimensions correspond to the dimensions of the screw canal from a metal screw previously used to treat a bone fracture.

4. The closure according to claims 1 to 3, characterized in that it is formed in such a way that it hermetically seals the medullary cavity in amputation stumps vis-à-vis internal pressure, and preferably takes the shape of a cap over the free end of a medullary pin.

5. The closure according to claims 1 to 4, characterized in that it comprises first to fourth order structures to facilitate bone formation, wherein the first order structure represents the outer shape which is adapted to the dimensions of the defect or hole to be closed off, the second order structure is formed in such a way that the elasticity of the closure increases continuously from the amputation stump to the medullary cavity so that its individual segments conform to the osseous system, and the third order structure representing the surface structure comprises individual shaping elements, each of which is in contact with the adjacent shaping elements, and which are arranged in one or more layers in contact with one another.

6. The closure according to claim 5, characterized in that the shaping elements are spheres and/or bulges or tubes with a diameter of between 200 and 3,000 µm, which are preferably microstructured on their surface, wherein the microstructure preferably consists of spherical segments or spheres with a diameter of 10 to 200 µm, preferably 15 to 30 µm, and/or of spheres in a composite with a matrix layer, wherein an organic material based on a collagen, polyglycolate, polylactate or a polypeptide is used as the matrix.

7. The closure according to claims 1 to 6, characterized in that it consists of an absorbable and/or non-absorbable organic and/or inorganic material.

8. The closure according to claims 5 to 7, characterized in that the shaping elements consist of tricalcium phosphate, apatite, collagen, a polypeptide or a mixture thereof and/or of a metal such as titanium, tantalum, cobalt, chromium, molybdenum or one of their alloys and/or of special steel.

9. The closure according to claims 1 to 8, characterized by a coating of inorganic fillers and an organic matrix, which preferably fills the spaces between the shaping elements and/or covers the surface of the closure, wherein the fillers of the coating are preferably spheres with a diameter of 10 to 200 µm, preferably 15 to 30 µm, and/or highly porous particles of tricalcium phosphate or hydroxyl apatite or particles of a related calcium compound or of bioglass, and wherein the matrix of the coating preferably consists of a polypeptide, collagen, polyglycolate or polylactate.

10. The closure according to claim 9, characterized in that the fillers of the coating partly take the form of fibres and/or form a closed network.

11. The closure according to any one of claims 1 to 10, characterized in that it and/or its coating consists of a material capable of swelling.

12. The closure according to claims 1 to 11, characterized in that an active pharmaceutical agent is included in the closure and/or its coating, e.g. an antibiotic such as gentamycin, and/or a cytostatic agent and/or bone morphogenetic protein and/or a haemostatic agent.

## Revendications

1. Fermeture, en un matériau chirurgical pour la fermeture de défauts ou de trous d'un os et/ou d'une cavité médullaire, qui est réalisée de façon qu'elle présente une forme telle et/ou de façon qu'elle soit formée en un matériau de sorte qu'elle s'adapte aux dimensions des défauts ou des trous de l'os et/ou aux dimensions de la cavité médullaire et de façon que par cela les défauts ou trous de l'os et/ou la cavité médullaire puissent être bouchés de façon étanche à la pression par rapport à la pression interne des canaux osseux et/ou de la cavité médullaire.

2. Fermeture suivant la revendication 1, caractérisée en ce qu'elle est réalisée sous la forme d'une vis (10), d'une cheville, d'un cône (40) d'un cylindre, d'un cylindre à lamelles (30), d'un couvercle, d'un bouchon (20), d'une fermeture par billes, d'un implant en corbeille ou d'un implant de cylindre creux, et/ou en ce que, dirigée de l'extérieur vers l'intérieur vers la cavité médullaire et/ou dirigée de l'intérieur vers l'extérieur à partir de la cavité médullaire, elle peut être appliquée, vissée et/ou bloquée.

3. Fermeture suivant la revendication 2, présentant la forme d'une vis (10), caractérisée en ce que ses dimensions correspondent aux dimensions du canal de vis d'une vis métallique utilisée précédemment pour le traitement d'une fracture d'os.

4. Fermeture suivant l'une quelconque des revendications 1 à 3, caractérisée en ce qu'elle est réalisée de façon qu'elle bouche hermétiquement la cavité médullaire de moignons d'amputation à l'encontre de pressions internes et en ce qu'elle est de préférence réalisée sous la forme d'une calotte au-dessus de l'extrémité libre d'un clou médullaire.

5. Fermeture suivant l'une quelconque des revendications 1 à 4, caractérisée en ce qu'elle présente pour la facilité de l'ossification une structure du premier au quatrième ordre, la structure du premier ordre représentant la forme externe qui est adaptée aux dimensions du défaut ou du trou à fermer, la structure du deuxième ordre étant réalisée de façon que l'élasticité de la fermeture augmente de façon continue depuis le moignon d'amputation vers la cavité médullaire de sorte que ses sections individuelles s'adaptent au squelette osseux, la structure du troisième ordre, représentant la structure superficielle, présentant des éléments de forme individuels qui sont respectivement en contact avec les éléments de forme voisins et qui sont agencés en une ou plusieurs couches restant en contact l'une avec l'autre.

6. Fermeture suivant la revendication 5, caractérisée en ce que les éléments de forme sont des billes et/ou des boudins ou des tubes d'un diamètre de 200 à 3000 µm qui, de préférence, présentent une microstructure à leur surface, la microstructure présentant de préférence des sections de bille ou des billes d'un diamètre de 10 à 200 µm, de préférence de 15 à 30 µm, et/ou étant constituée par des billes en liaison avec une couche de matrice, un matériau organique sur base d'un collagène, d'un polyglycolate, d'un polylactate ou d'un polypeptide étant utilisé comme matrice.

7. Fermeture suivant l'une quelconque des revendications 1 à 6, caractérisée en ce qu'elle est constituée par un matériau organique et/ou inorganique résorbable et/ou non résorbable.

8. Fermeture suivant l'une quelconque des revendications 5 à 7, caractérisée en ce que les éléments de forme sont constitués par du phosphate tricalcique, de l'apatite, du collagène, un polypeptide ou un mélange de ceux-ci et/ou par un métal comme du titane, du tantale, du cobalt, du chrome, du molybdène ou par un de leurs alliages et/ou par un acier spécial.

9. Fermeture suivant l'une quelconque des revendications 1 à 8, caractérisée par une enduction qui est constituée par des corps de remplissage inorganiques et par une matrice organique et qui remplit de préférence les espaces intermédiaires entre les éléments de forme et/ou qui recouvre la surface de la fermeture, les corps de remplissage de l'enduction étant de préférence des billes d'un diamètre de 10 à 200 µm, de préférence de 15 à 30 µm, et/ou des particules à porosité élevée en phosphate tricalcique ou en apatite hydroxylée ou des particules à base d'un composé calcique apparenté ou en bioverre, la matrice de l'enduction étant de préférence constituée par un polypeptide, un collagène, un polyglycolate ou un polylactate.

10. Fermeture suivant la revendication 9, caractérisée en ce que les corps de remplissage de l'enduction ont partiellement une forme de fibre et/ou forment un réseau fermé.

11. Fermeture suivant l'une quelconque des revendications 1 à 10, caractérisée en ce qu'elle-même et/ou son enduction sont constituées par un matériau susceptible de gonfler.

12. Fermeture suivant l'une quelconque des revendications 1 à 11, caractérisée en ce que dans la fermeture et/ou dans son enduction est contenu un additif pharmaceutique, par exemple un antibiotique comme de la gentamicine, et/ou un cytostatique et/ou une protéine morphogénétique d'os (Bone morphogenetic Protein) et/ou un hémostyptique.

FIG.4

44

46

42    40

48

30

32

FIG.3

20a    20b

FIG 2

10

FIG 1